# EUROPÄISCHE PATENTSCHRIFT

(11) **EP 0 713 862 B1**
(45) Veröffentlichungstag und Bekanntmachung des Hinweises auf die Patenterteilung: **16.09.1998**
(21) Anmeldenummer: 95117655.1
(22) Anmeldetag: 09.11.1995
(51) Int. Cl.: C07C 255/50, A01N 37/34, C07C 253/20, C07C 253/00, C07C 251/48, C07C 233/65, C07C 25/13, C07C 63/70, C07C 47/55

(54) **Herbizide Mittel auf Basis von 2,6-Dichlor-3-fluor- benzonitril.**
Herbicidal substance based on 2,6-dichloro-3-fluoro- benzonitrile.
Agent herbicidal basé sur 2,6-dichloro-3-fluoro- benzonitrile.

(30) Priorität: 22.11.1994 DE 4441419
(43) Veröffentlichungstag der Anmeldung: 29.05.1996
(60) Teilanmeldung: 98102479.7
(73) Patentinhaber: BAYER AG, 51368 Leverkusen (DE)
(72) Erfinder: Lantzsch, Reinhard, Dr., D-42115 Wuppertal (DE); Bussmann, Werner, Dr., D-51373 Leverkusen (DE); Käsbauer, Josef, Dr., D-42929 Wermelskirchen (DE); Dollinger, Markus, Dr., D-51381 Leverkusen (DE); Santel, Hans-Joachim, Dr., D-51371 Leverkusen (DE)

(56) Entgegenhaltungen:
- EP-A- 0 273 317
- EP-A- 0 599 241
- EP-A- 0 609 179
- EP-A- 0 657 407
- FR-A- 1 232 614
- CHEMICAL ABSTRACTS, vol. 115, no. 17, 28.Oktober 1991 Columbus, Ohio, US; abstract no. 182866k, I. SHIGEHARA ET. AL.: "Preparation of 2,6-dichloro-3-fluorobenzonitrile" Seite 878; Spalte 2; XP002006423 & JP-A-03 900 057 (ISHIHARA SANGYO KAISHA LTD.)

## Beschreibung

Die Erfindung betrifft die Verwendung der bekannten Verbindung 2,6-Dichlor-3-fluor-benzonitril als Wirkstoff in selektiv herbiziden Mitteln.

2,6-Dichlor-3-fluor-benzonitril ist bereits als Zwischenprodukt für die Herstellung von 2,3,6-Trifluor-benzonitril bekannt geworden (vgl. JP-A 03090057 - zitiert in Chem. Abstracts 115:182866). Die Verwendung der genannten Verbindung als Herbizid ist jedoch bisher nicht bekannt geworden. Bezüglich der Herstellung wird in der zitierten Literatur angegeben, daß zunächst 3-Chlor-2,4-difluor-nitrobenzol mit einem Alkalimetallcyanid unter Bildung von 2-Chlor-3-fluor-6-nitro-benzonitril umgesetzt wird, welches dann durch Umsetzung mit Chlor in 2,6-Dichlor-3-fluor-benzonitril umgewandelt wird. Ausbeute und Qualität des so erhaltenen Produktes sind hierbei nicht immer ganz zufriedenstellend.

In EP-A 0 273 317 ist erwähnt, daß 2,6-Dichlor-benzonitril als Herbizid industrielle Bedeutung erlangt hat.

Es wurde nun gefunden, daß die Verbindung 2,6-Dichlor-3-fluor-benzonitril der nachstehenden Formel (I) 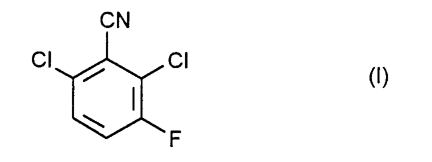 sehr gut zur selektiven Bekämpfung von Unkräutern in wichtigen Kulturen verwendet werden kann.

Die Verbindung der Formel (I) wird erhalten, wenn man 2,6-Dichlor-3-fluor-benzaldoxim der Formel (II) oder 2,6-Dichlor-3-fluor-benzamid der Formel (III) 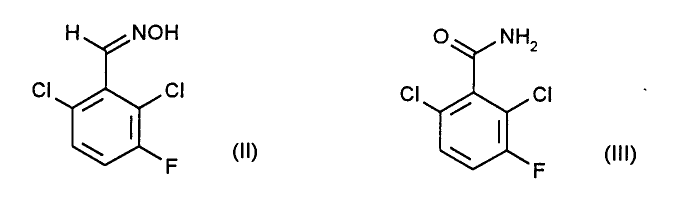 mit einem wasser-entziehenden (dehydratisierenden) Mittel bei Temperaturen zwischen 0°C und 300°C im Druckbereich zwischen 0,001 bar und 10 bar umsetzt (vgl. die Herstellungsbeispiele).

Die erfindungsgemäß zu verwendende Verbindung der Formel (I) kann nach diesem Herstellungsverfahren wesentlich einfacher und auf Basis gut zugänglicher Ausgangsstoffe in sehr guten Ausbeuten hergestellt werden.

Man erhält die neue Verbindung der Formel (II), wenn man 2,6-Dichlor-3-fluor-benzaldehyd der Formel (IV) 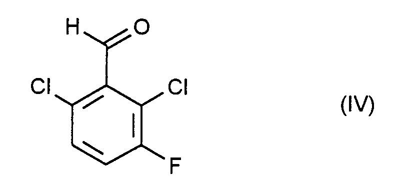 mit Hydroxylamin oder einem Säureaddukt hiervon, wie z.B. Hydroxylamin-Hydrochlorid, gegebenenfalls in Gegenwart eines Verdünnungsmittels, wie z.B. Methanol, Ethanol und/oder Wasser, und gegebenenfalls in Gegenwart eines Säureakzeptors, wie z.B. Natriumacetat, bei Temperaturen zwischen 0°C und 100°C umsetzt (vgl. die Herstellungsbeispiele).

Man erhält die neue Verbindung der Formel (III), wenn man 2,6-Dichlor-3-fluor-benzoesäure der Formel (V) 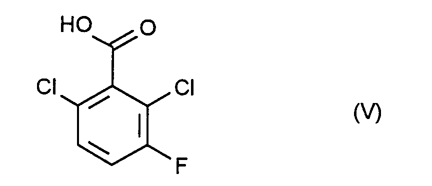 mit einem Chlorierungsmittel, wie z.B. Thionylchlorid, gegebenenfalls in Gegenwart eines Reaktionshilfsmittels, wie z.B. N,N-Dimethyl-formamid, bei Temperaturen zwischen 0°C und 100°C umsetzt und das hierbei erhaltene 2,6-Dichlor-3-fluor-benzoesäurechlorid mit Ammoniak, gegebenenfalls in Gegenwart eines Verdünnungsmittels, wie z.B. Wasser, bei Temperaturen zwischen -50°C und +50°C umsetzt (vgl. die Herstellungsbeispiele).

Man erhält die Verbindung der Formel (IV), wenn man 2,6-Dichlor-3-fluor-benzalchlorid der Formel (VI) 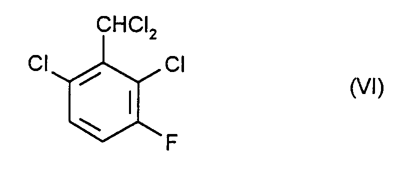 mit Wasser, gegebenenfalls in Gegenwart eines Reaktionshilfsmittels, wie z.B. Hydrogenchlorid und/oder Eisen(III)-chlorid, bei Temperaturen zwischen 0°C und 200°C umsetzt (vgl. die Herstellungsbeispiele).

Man erhält die neue Verbindung der Formel (VI), wenn man 2,6-Dichlor-3-fluor-toluol der Formel (VII) 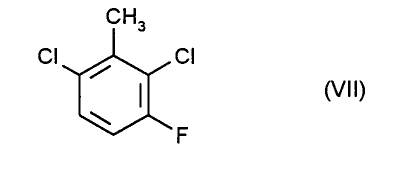 mit Chlor, gegebenenfalls in Gegenwart eines Reaktionshilfsmittels, wie z.B. Pyridin, bei Temperaturen zwischen 0°C und 100°C umsetzt (vgl. die Herstellungsbeispiele).

Man erhält die Verbindung der Formel (VII), wenn man 3-Fluor-toluol der Formel (VIII) 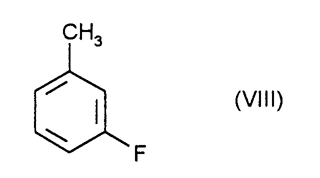 mit Chlor, gegebenenfalls in Gegenwart eines Reaktionshilfsmittels, wie z.B. Eisen(III)-chlorid und 2,3-Dihydro-5H-benzo[b]-1,4-thiazepin-4-on bei Temperaturen zwischen 0°C und 100°C umsetzt (vgl. die Herstellungsbeispiele).

3-Fluor-toluol ist eine handelsübliche Synthesechemikalie.

Man erhält die neue Verbindung der Formel (V), wenn man 2,6-Dichlor-3-fluor-benzaldehyd der Formel (IV) -oben - mit einem Oxidationsmittel, wie z.B. Hydrogenperoxid, gegebenenfalls in Gegenwart eines Reaktionshilfsmittels, wie z.B. Natriumhydroxid, und gegebenenfalls in Gegenwart eines Verdünnungsmittels, wie z.B. Wasser, bei Temperaturen zwischen 0°C und 100°C umsetzt (vgl. die Herstellungsbeispiele).

Das Verfahren zur Herstellung der Verbindung der Formel (I) wird unter Verwendung eines wasser-entziehenden (dehydratisierenden) Mittels durchgeführt. Es kommen hierbei die üblichen zur Dehydratisierung von Aldoximen geeigneten Mittel in Betracht. Als Beispiele hierfür seien Acetanhydrid, Thionylchlorid, Phosphor(V)-oxid, Dicyclohexylcarbodiimid, Cyanurchlorid, Titan(IV)-chlorid und Benzolsulfonsäurechlorid genannt.

Die Reaktionstemperaturen können bei der Durchführung des Verfahrens zur Herstellung der Verbindung der Formel (I) in einem größeren Bereich variiert werden. Im allgemeinen arbeitet man bei Temperaturen zwischen 0°C und 300°C, vorzugsweise bei Temperaturen zwischen 20°C und 250°C, insbesondere bei Temperaturen zwischen 50°C und 200°C.

Das Verfahren zur Herstellung der Verbindung der Formel (I) wird im allgemeinen unter Normaldruck durchgeführt. Es ist jedoch auch möglich, unter erhöhtem oder vermindertem Druck - im allgemeinen zwischen 0,001 bar und 10 bar, vorzugsweise zwischen 0,01 bar und 5 bar, insbesondere zwischen 0,1 bar und 2 bar - zu arbeiten.

Bei der Durchführung des Verfahrens zur Herstellung der Verbindung der Formel (I) setzt man im allgemeinen auf 1 Mol Ausgangsverbindung der Formel (II) oder der Formel (III) wenigstens 1 Mol eines wasser-entziehenden Mittels ein. Das Wasser-entziehende Mittel kann - vorzugsweise bei Einsatz von Acetanhydrid - auch in hohem Überschuß und damit auch als Verdünnungsmittel verwendet werden. Die Umsetzung und die Aufarbeitung erfolgen nach üblichen Methoden (vgl. die Herstellungsbeispiele).

Der erfindungsgemäß zu verwendende Wirkstoff kann als Defoliant, Desiccant, Krautabtötungsmittel und insbesondere als Unkrautvernichtungsmittel eingesetzt werden. Unter Unkraut im weitesten Sinne sind alle Pflanzen zu verstehen, die an Orten aufwachsen, wo sie unerwünscht sind. Ob der erfindungsgemäß zu verwendende Wirkstoff als totales oder selektives Herbizid wirkt, hängt im wesentlichen von der angewandten Menge ab.

Der erfindungsgemäß zu verwendende Wirkstoff kann z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbrisrylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung des erfindungsgemäßen Wirkstoffs ist jedoch keineswegs auf diese Gattungen beschränkt, sondern erstreckt sich in gleicher Weise auch auf andere Pflanzen.

Der Wirkstoff der Formel (I) eignet sich in Abhängigkeit von der Konzentration zur Totalunkrautbekämpfung z.B. auf Industrie- und Gleisanlagen und auf Wegen und Plätzen mit und ohne Baumbewuchs. Ebenso kann die Verbindung zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäß zu verwendende Verbindung der Formel (I) eignet sich insbesondere zur selektiven Bekämpfung von monokotylen und dikotylen Unkräutern in monokotylen und dikotylen Kulturen sowohl im Vorauflauf- als auch im Nachauflauf-Verfahren.

Der Wirkstoff kann in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Fertocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Der erfindungsgemäß zu verwendende Wirkstoff kann als solcher oder in seinen Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Der Wirkstoff kann als solcher, in Form seiner Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Der erfindungsgemäß zu verwendende Wirkstoff kann sowohl vor, als auch nach dem Auflaufen der Pflanzen appliziert werden. Er kann auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung des erfindungsgemäßen Wirkstoffs geht aus den nachfolgenden Beispielen hervor.

### Herstellungsbeispiele:

### Beispiel 1

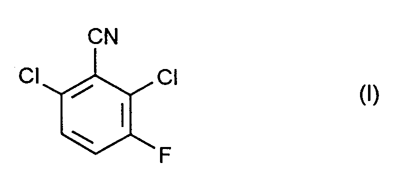

80 g (0,385 Mol) 2,6-Dichlor-3-fluor-benzaldoxim der Formel (II) werden mit 500 ml Acetanhydrid bei Raumtemperatur (ca. 20°C) vermischt. Es bildet sich eine klare Lösung, die dann 6 Stunden lang bei 135°C gerührt wird. Nach Abdestillieren von Essigsäure und Acetanhydrid wird der Rückstand auf Raumtemperatur abgekühlt und in Methylenchlorid aufgenommen. Die Lösung wird dreimal mit Wasser gewaschen, dann mit Natriumsulfat getrocknet und filtrieren Vom Filtrat wird dann das Lösungsmittel unter vermindertem Druck sorgfältig abdestilliert.

Man erhält 67 g (91% der Theorie) 2,6-Dichlor-3-fluor-benzonitril als kristallinen Rückstand vom Schmelzpunkt 69°C-71°C.

### Beispiel 2

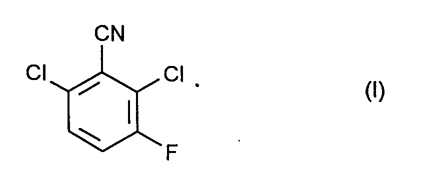

Eine Mischung aus 2,08 g (0,01 Mol) 2,6-Dichlor-3-fluor-benzamid und 1,6 g (0,011 Mol) Phosphor(V)-oxid wird in einer kleinen Destillationsapparatur bei einem Druck von ca. 100 bar auf ca. 200°C erhitzt.

Man erhält so 1,4 g (74% der Theorie) 2,6-Dichlor-3-fluor-benzonitril vom Schmelzpunkt 70°C-72°C.

### Ausgangsverbindung der Formel (II):

### Beispiel (II-1)

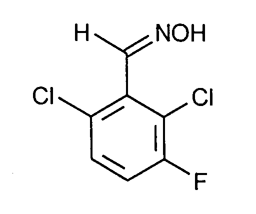

78,3 g (0,383 Mol) 2,6-Dichlor-3-fluor-benzaldehyd (94,5%ig) - in 150 ml Methanol gelöst - werden zu einer Lösung von 31 g (0,446 Mol) Hydroxylaminhydrochlorid und 36,6 g (0,446 Mol) Natriumacetat in 400 ml Methanol und 40 ml Wasser getropft. Die Reaktion ist exotherm (32°C). Man rührt zwölf Stunden bei Raumtemperatur (20°C) nach und filtriert ab. Der Feststoff wird zweimal mit Wasser ausgerührt und abfiltriert. Schmelzpunkt: 130°C-131°C. Durch Einengen der Mutterlauge und Ausfällen mit Wasser kann weiteres Reaktionsprodukt erhalten werden.

Gesamtausbeute: 80 g (94,8%iges Produkt); Ausbeute: 94% der Theorie.

### Ausgangsverbindung der Formel (III):

### Beispiel (III-1)

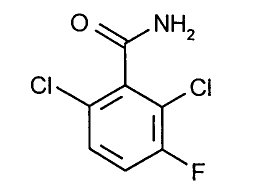

20,9 g (0,1 Mol) 2,6-Dichlor-3-fluor-benzoesäure werden mit 100 ml Thionylchlorid und einigen Tropfen Dimethylformamid 3 Stunden zum Sieden erhitzt. Der nach Abdestillieren des überschüssigen Thionylchlorids hinterbleibende Rückstand wird zu 300 ml konzentrierter Ammoniaklösung getropft. Man filtriert ab, wäscht mit Wasser und trocknet.

Ausbeute: 20,4 g (98% der Theorie) 2,6-Dichlor-3-fluor-benzamid vom Schmelzpunkt 183°C-186°C.

### Ausgangsverbindung der Formel (IV):

### Beispiel (IV-1)

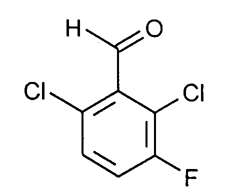

200 g (0,85 Mol) 2,6-Dichlor-3-fluor-benzalchlorid werden in einem Vierhalskolben mit Rührer, Rückflußkühler, Thermometer und Tropftrichter vorgelegt. Dann werden 200 mg Eisen(III)-chlorid - in 23 ml Salzsäure (32%ig) gelöst - innerhalb von 4 Stunden bei 160°C zugetropft. Es wird ein Rohaldehyd mit weniger als 0,7% des Benzalchlorids erhalten.

Nach Destillation im Vakuum (20 mbar) werden 120 g reiner 2,6-Dichlor-3-fluor-benzaldehyd (98,5%ig) erhalten, Ausbeute: 77% der Theorie.

### Ausgangsverbindung der Formel (V):

### Beispiel (V-1)

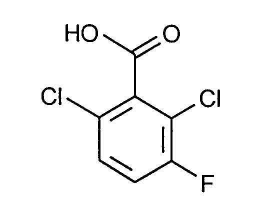

11,6 g (0,06 Mol) 2,6-Dichlor-3-fluor-benzaldehyd (94,5%ig) wird in 30 ml Wasser bei 60°C aufgeschmolzen und unter starkem Rühren mit 1 ml 30%iger Natronlauge versetzt.

Anschließend werden 29,1 g (0,3 Mol) Wasserperoxid (35%ig) und 12 g (0,09 Mol) Natronlauge (30%ig) aus zwei Tropftrichtern simultan zudosiert. Die Reaktionstemperatur steigt dabei auf 70°C. Man hält noch eine Stunde bei dieser Temperatur und läßt dann die klare Reaktionslösung abkühlen. Man extrahiert zweimal mit Methylenchlorid und stellt dann auf pH 1. Man filtriert ab, wäscht mit Wasser und trocknet. Ausbeute: 10,5 g (83% der Theorie); Schmelzpunkt: 127°C-131°C.

### Ausgangsverbindung der Formel (VI):

### Beispiel (VI-1)

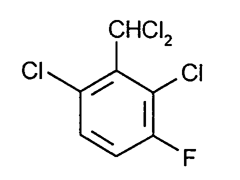

360 g Dichlorfluortoluol-Isomerengemisch (50% 2,6-Dichlor-3-fluor-toluol) werden vorgelegt. Nach Zugabe von 1 Tropfen Pyridin wird Chlor eingeleitet, bis das 2,6-Isomere vollständig in 2,6-Dichlor-3-fluor-benzalchlorid umgewandelt ist. Es liegt dann ein Gemisch mit der ungefähren Zusammensetzung 45% 2,6-Dichlor-3-fluor-benzalchlorid, 45% 4,6-Dichlor-3-fluor-benzalchlorid, 5% 4,6-Dichlor-3-fluor-benzotrichlorid vor.

Dieses wird durch fraktionierte Destillation getrennt. Hierbei wird ein Hauptlauf mit 98,5%igem 2,6-Dichlor-3-fluor-benzalchlorid erhalten, das direkt in die Hydrolysereaktion eingesetzt wird; Ausbeute: 200 g (80% der Theorie).

### Ausgangsverbindung der Formel (VII):

### Beispiel (VII-1)

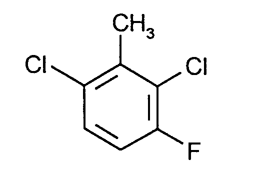

330 g 3-Fluor-toluol werden in einem Vierhalskolben mit Rührer, Rückflußkühler, Thermometer, Gaseinleitungsrohr vorgelegt. Nach Zugabe von 2,3 g Eisen(III)-chlorid und 450 mg 2,3-Dihydro-5H-benzo[b]-1,4-thiazepin-4-on wird bei 20°C unter Rühren Chlor eingeleitet bis nur noch 1 Gewichtsprozent des Mono-chlorderivates vorhanden ist. Es wird ein Gemisch mit 43% 4,6-Dichlor-3-fluor-toluol und 43 Gewichtsprozent 2,6-Dichlor-3-fluor-toluol erhalten.

Das Reaktionsgemisch wird zweimal mit Wasser (jeweils 60 ml) gewaschen und anschließend destilliert. Dabei wird ein Vorlauf (60 g) erhalten, der im wesentlichen Monochlorfluortoluol enthält und dem nächsten Ansatz zugeführt wird. Als Hauptlauf werden 360 g des Isomeren-Gemisches erhalten, der verbleibende Rückstand wird ebenfalls wieder eingesetzt.

Nach fünffacher Wiederholung liegt die Ausbeute bei 80% der Theorie.

Das erhaltene Isomerengemisch aus 50% 2,6- und 50% 4,6-Dichlor-3-fluor-toluol wird direkt in die nächste Stufe eingesetzt.

### Anwendungsbeispiele:

### Beispiel A

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.
Es bedeuten:
- 0 % =: keine Wirkung (wie unbehandelte Kontrolle)
- 100 % =: totale Vernichtung

In diesem Test zeigt die Verbindung der Formel (I) bei einer Aufwandmenge von 2000g/ha sehr gute Verträglichkeit gegenüber Kulturpflanzen, wie z.B. Weizen und Soja (jeweils 10 %) und starke Wirkung gegen Unkräuter wie Avena (80 %) und Galium (80 %).

### Beispiel B

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
- 0 % =: keine Wirkung (wie unbehandelte Kontrolle)
- 100 % =: totale Vernichtung

In diesem Test zeigt die Verbindung der Formel (I) bei einer Aufwandmenge von 500 g/ha starke Wirkung gegen Unkräuter wie Digitaria (100 %), Lolium (100 %), Panicum (100 %), Chenopodium (100 %), Galinsoga (100 %), Ipomoea (100 %) und Stellaria (100 %).

## Patentansprüche

1. Herbizide Mittel, gekennzeichnet durch einen Gehalt an 2,6-Dichlor-3-fluor-benzonitril der Formel (I)

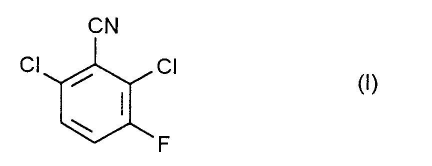

2. Verfahren zur Bekämpfung von unerwünschten Pflanzen, dadurch gekennzeichnet, daß man 2,6-Dichlor-3-fluor-benzonitril auf unerwünschte Pflanzen und/oder ihren Lebensraum einwirken läßt.

3. Verwendung von 2,6-Dichlor-3-fluor-benzonitril zur Bekämpfung von unerwünschten Pflanzen.

4. Verfahren zur Herstellung von herbiziden Mitteln, dadurch gekennzeichnet, daß man 2,6-Dichlor-3-fluor-benzonitril mit Streckmitteln und/oder oberflächenaktiven Substanzen vermischt.

## Claims

1. Herbicidal compositions, characterized in that they comprise 2,6-dichloro-3-fluoro-benzonitrile, of the formula (I)

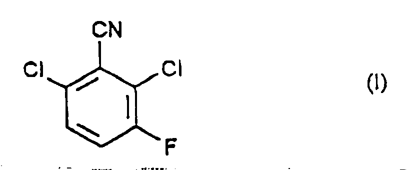

2. Method of combating undesirable plants, characterized in that 2,6-dichloro-3-fluoro-benzonitrile is allowed to act on undesirable plants and/or their environment.

3. Use of 2,6-dichlor-3-fluorobenzonitrile for combating undesirable plants.

4. Process for the preparation of herbicidal compositions, characterized in that 2,6-dichloro-3-fluoro-benzonitrile is mixed with extenders and/or surface-active substances.

## Revendications

1. Compositions herbicides, caractérisées par une teneur en 2,6-dichloro-3-fluorobenzonitrile de formule (I)

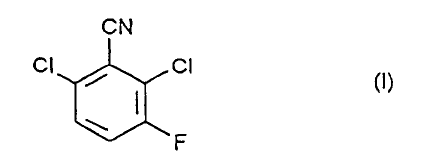

2. Procédé pour combattre des plantes indésirables, caractérisé en ce qu'on fait agir du 2,6-dichloro-3-fluorobenzonitrile sur des plantes indésirables et/ou sur leur milieu.

3. Utilisation du 2,6-dichloro-3-fluorobenzonitrile pour combattre des plantes indésirables.

4. Procédé de préparation de compositions herbicides, caractérisé en ce gu'on mélange du 2,6-dichloro-3-fluorobenzonitrile avec des diluants et/ou des agents tensio-actifs.
